(19) Europäisches Patentamt
European Patent Office
Office européen des brevets

(11) Veröffentlichungsnummer : **0 112 517**
**B1**

(12) # EUROPÄISCHE PATENTSCHRIFT

(45) Veröffentlichungstag der Patentschrift : **16.04.86**

(51) Int. Cl.⁴ : **C 07 H 19/00**, A 61 K 31/70, A 23 K 1/16

(21) Anmeldenummer : **83111971.4**

(22) Anmeldetag : **29.11.83**

(54) **Polyätherverbindungen.**

(30) Priorität : **29.11.82 US 444958**
**24.10.83 US 530187**

(43) Veröffentlichungstag der Anmeldung : **04.07.84 Patentblatt 84/27**

(45) Bekanntmachung des Hinweises auf die Patenterteilung : **16.04.86 Patentblatt 86/16**

(84) Benannte Vertragsstaaten : **AT BE CH DE FR GB IT LI LU NL SE**

(56) Entgegenhaltungen : **EP-A- 0 035 119**

(73) Patentinhaber : **F. HOFFMANN-LA ROCHE & CO. Aktiengesellschaft**

**CH-4002 Basel (CH)**

(72) Erfinder : **Foley, Louise H.**
**30 Park Court**
**Durham, New Hampshire (US)**
Erfinder : **Westley, John**
**91 South Mountain Avenue**
**Cedar Grove, New Jersey (US)**
Erfinder : **Sello, Lillan H.**
**43 Reservoir Place**
**Cedar Grove, New Jersey (US)**

(74) Vertreter : **Lederer, Franz, Dr. et al**
**Patentanwälte Dr. Lederer Franz Meyer-Roxlau Reiner F. Lucile-Grahn-Strasse 22**
**D-8000 München 80 (DE)**

**EP 0 112 517 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft ein Verfahren zur Herstellung von Aethern des Antibiotikums X-14868A der Formel

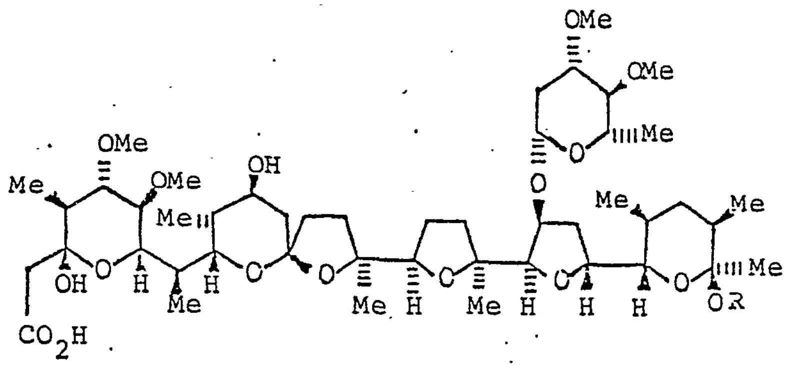

(I)

worin R $C_1$-$C_7$-Alkyl oder $C_2$-$C_7$-Hydroxyalkyl bedeutet, und der pharmazeutisch annehmbaren Säureadditionssalze davon sowie die neuen Verbindungen der obigen Formel I, in der R $C_2$-$C_7$-Alkyl oder $C_2$-$C_7$-Hydroxyalkyl bedeutet, und deren pharmazeutisch annehmbare Säureadditionssalze.

Der Ausdruck $C_1$-$C_7$-Alkyl beudeutet einen geradkettigen oder verzweigten Kohlenwasserstoffrest mit 1-7 C-Atomen, vorzugsweise 2-6 C-Atomen, wie Aethyl, Butyl, Hexyl und dergleichen.

Die Abkürzung « Me » in der obigen Formel bedeutet Methyl.

Die Verbindung X-14868A ist eine bekannte Verbindung, welche in U.S.-Patentschrift No. 4,278,663, welche am 14. Juli 1981 veröffentlicht wurde, beschrieben wird siehe auch EP-A-35119). In dieser Patentschrift wird ebenfalls ein Verfahren zur Herstellung des Antibiotikums unter Verwendung von Nocardia sp. X-14868A beschrieben, wobei Nocardia sp. X-14868 bei der American Type Culture Collection, Rockville, Maryland unter der Nummer ATCC 31585 hinterlegt wurde und für das Publikum erhältlich ist.

Die erfindungsgemäßen neuen Verbindungen haben ähnliche Wirkungen wie die in EP-A-35 119 beschreibenen. Sie sind außerdem als Antimalariamittel verwendbar.

Die Verbindungen der Formel I, und zwar sowohl die bekannte Verbindung der Formel I, worin R Methyl bedeutet, als auch die neuen Verbindungen, worin R $C_2$-$C_7$-Alkyl oder $C_2$-$C_7$-Hydroxyalkyl bedeutet, werden hergestellt durch Reaktion von X-14868A der Formel

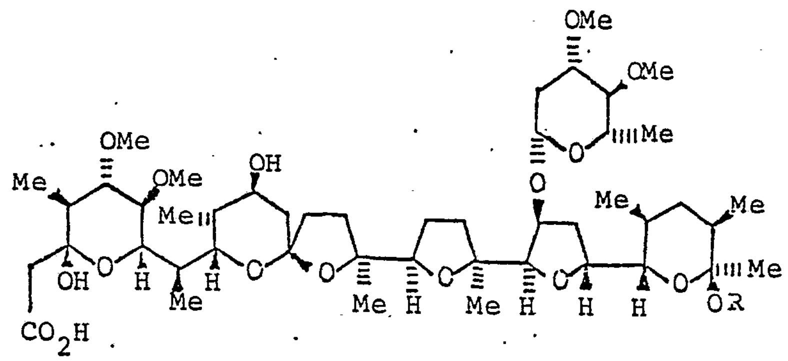

mit einem primären oder sekundären Alkohol mit 1-7 C-Atomen oder einem difunktionellen Alkohol, d. h. einem Diol mit 2-7 C-Atomen in Gegenwart eines sauren Ionenaustauscherharzes in der $H^+$-Form, wie ein Sulfonsäureharz in der $H^+$-Form wie z. B. DOWEX 50W in der $H^+$-Form. Die Reaktion wird unter Verwendung des Alkohols oder Diols als Lösungsmittel oder in einem inerten Lösungsmittel bei ungefähr Umgebungstemperatur, z. B. Raumtemperatur durchgeführt.

Beispiele geeigneter Alkohole umfassen geradkettige oder verzweigte Alkohole mit 1-7 C-Atomen, wie z. B. Aethanol, n- und Isopropanol, n-Butanol, n-Hexanol und dergleichen. Beispiele von geeigneten Diolen umfassen die Difunktionellen Alkohole mit 2-7 C-Atomen, wie z. B. Aethylenglykol, n-Butylenglykol und dergleichen.

Sofern Alkohole verwendet werden, die sowohl in der (R)- wie in der (S)-Konfiguration vorliegen können, werden beide Konfigurationen von der vorliegenden Erfindung umfasst.

Die Verbindungen gemäss vorliegender Erfindung zeigen Aktivität als anticoccidiostatische Mittel. So zeigen z. B. der n-Propyläther und der Aethyläther des Antibiotikums X-14868A eine Aktivität von ungefähr 20 bis ungefähr 35 ppm beim Test in zwei Wochen alten Batterieküken gegen gemischte Eimeria Feldisolate. Der Test besteht aus dem Routinelaborverfahren Coccidiose. Es werden uninfizierte unbehandelte Kontrollen (UUC) und infizierte unbehandelte Kontrollen (IUC) verwendet. Die Küken werden zwei Tage vor der Infektion während 8 aufeinanderfolgenden Tagen medikamentös behandelt.

Die Küken werden mit 40 000 gemischten Oocyten von E. acervulina, E. mivati, E. maxima, E. necatrix und E. tenella Feldstämmen infiziert. Es werden 10 Küken pro Gruppe verwendet. Die Ergebnisse sind in der nachfolgenden Tabelle wiedergegeben :

| | Konz. in Futter, ppm | Gewichts-zunahme, % | Morta-lität % | Verletzungszahl Obere | Mittlere | cekale | Durchschnitt |
|---|---|---|---|---|---|---|---|
| UUC | 0 | 100 | 0 | 0.0 | 0.0 | 0.0 | 0.0 |
| IUC | 0 | 48 | 40 | 3.1 | 3.0 | 3.2 | 3.1 |
| Aethyläther von X-14868A | 35 | 73 | 0 | 0.0 | 0.0 | 0.0 | 0.0 |
| | 25 | 75 | 0 | 0.5 | 0.0 | 0.0 | 0.2 |
| | 20 | 81 | 0 | 1.5 | 1.5 | 1.6 | 1.5 |
| n-Propyläther von X-14868A | 35 | 55 | 0 | 0.0 | 0.0 | 0.0 | 0.0 |
| | 25 | 87 | 0 | 0.1 | 0.0 | 0.0 | 0.03 |
| | 20 | 72 | 0 | 1.5 | 1.5 | 1.4 | 1.5 |

Die Verbindungen der vorliegenden Erfindung zeigen Aktivität als Antimalariamittel. Der Aethylenglycol, n-Hexyl, Aethyl, n-Butyl und n-Propyläther von X-14868A wurden gegen Chloroquin und Pyrimethamin getestet, welch letztere bekannte Antimalariamittel sind. Die Verbindungen wurden zweimal in einem in-vitro Screeningtest gegen zwei Stämme von Plasmodium falciparum getestet. Die Aktivität ($ID_{50}$) wird in µg/l ausgedrückt. Die Ergebnisse werden in zwei Werten wie folgt ausgedrückt :

| Verbindung | Plasmodium falciparum Stamm 13 | T9 Clon 96 |
|---|---|---|
| Chloroquin | 390- 550 | 44- 75 |
| Pyrimithamin | 55- 61 | 68- 70 |
| n-Propyläther von X-14868A | 300- 840 | 560-450 |
| Aethyläther von X-14868A | 142- 270 | 205-145 |
| n-Butyläther von X-14868A (Natriumsalz) | 500-1200 | 1150-830 |
| Aethylenglycoläther von X-14868A (Natriumsalz) | 39- 76 | 48- 39 |
| n-Hexyläther von X-14868A (Natriumsalz) | | 930-650 |
| n-Pentyläther von X-14868A (Natriumsalz) | | 1600-800 |

Zur Evaluation der anticoccidiostatischen Wirksamkeit des 1,3-Propandioläthers des Antibiotikums X-14868A wurde eine weitere Studie gegen gemischte Eimeria Spezies in 2 Wochen alten Batterieküken durchgeführt. Die in dieser Studie verwendeten Eimeria-Stämme wurden aus kommerziell erhältlichen Küken gewonnen, welche in früheren Studien nicht verwendet wurden. Für Vergleichzwecke wurden die Antibiotika Monensin, Lasolocid und das Antibiotikum X-14868A verwendet. 2 Wochen alte Hubbard Cross-Küken, welche kommerziell von einer Brutanstalt erhältlich waren, und welche in einer elektrisch geheizten Batterie mit Drahtboden gehalten wurden, werden in allen Studien verwendet. 10 Küken ausgewählt nach Gewicht und Geschlecht (50 % weiblich und 50 % männlich) werden in je drei identischen Gruppen verwendet. Die Küken werden 2 Tage vor Infektion medikamentös behandelt und bis zur Beendigung des Versuchs, d. h. 6 Tage nach der Infektion unter Antibiotika gehalten. Für jedes Experiment werden uninfizierte, unbehandelte Kontrollen und infizierte, unbehandelte Kontrollen verwendet.

Als Küken-Startgemisch wurde eine Basisration einer kompletten Futterzusammensetzung verwendet, welche frei von Medikamenten war. Medikament enthaltendes Futter wurde durch Zugabe der gewünschten Konzentration an Arzneimittel zur Basisration hergestellt. Jede Arzneimittelkonzentration

wurde sorgfältig vor der Verwendung in das Futtermittelgemisch gemischt, um eine einheitliche Mischung zu erhalten. In jedem Fall wurde das Medikament enthaltende Futter 2 Tage vor der Infektion und für eine Gesamtdauer von 8 nacheinanderfolgenden Tagen verfüttert.

Die Infektion wurde oral induziert, und zwar durch direkte Inokulation einer Suspension enthaltend E. acervullna/E. mivati — 500 000, E. maxima — 100 000, E. tenella — 100 000 und E. brunetta — 50 000 sporulierter Oocysten, welche gut geschüttelt und in sterilem destilliertem Wasser in einer Menge von 1 ml suspendiert sind, in den Kropf der Tiere mit Hilfe einer stumpfen Nadel, welche mit einer kalibrierten Injektionsspritze verbunden ist.

Nach Beendigung des Versuches werden die überlebenden Küken getötet und zur Untersuchung auf grobe Verletzungen autopsiert. Autopsiert werden auch alle Küken, die während des Experiments gestorben sind. Die Diagnose basierte aufgrund des Ortes und der Morphologie der Verletzung. Die Ergebnisse werden als durchschnittlicher Infektionsgrad (ADI) gemäss nachfolgendem Zählsystem angegeben : 0 = normal, 1 = leicht, 2 = mässig, 3 = schwer und 4 = tot.

Zudem wurde die Gewichtszunahme (%) aufgezeichnet.

| Antibiotikum | Konz. im Futter,ppm | Gewichts-zunahme % | Obere | Mittlere | cecale | ADI |
|---|---|---|---|---|---|---|
| UUC | 0 | 100 | 0.0 | 0.0 | 0.0 | 0.0 |
| IUC | 0 | 50 | 2.7 | 2.4 | 2.8 | 2.6 |
| 1,3-Propandioläther von X-14868A | 15 | 71 | 0.0 | 0.0 | 0.0 | 0.0 |
| | 12.5 | 81 | 0.0 | 0.0 | 0.07 | 0.02 |
| | 10 | 96 | 0.1 | 0.03 | 0.1 | 0.1 |
| | 7.5 | 91 | 0.7 | 0.3 | 0.5 | 0.5 |
| | 5 | 82 | 1.0 | 0.9 | 1.1 | 1.0 |
| | 2.5 | 59 | 2.3 | 2.1 | 2.2 | 2.2 |
| Antibiotikum X-14868A | 10 | 86 | 0.1 | 0.07 | 0.2 | 0.1 |
| | 7.5 | 100 | 0.4 | 0.2 | 0.6 | 0.4 |
| | 5 | 83 | 1.3 | 1.1 | 1.2 | 1.2 |
| Lasalocid | 100 | 99 | 0.5 | 0.5 | 0.9 | 0.6 |
| Monensin | 100 | 80 | 1.1 | 1.1 | 2.0 | 1.4 |

Die Verabfolgung der Antibiotikaäther von X-14868A nachfolgend « Antibiotika » oder « antibiotische Verbindungen » genannt, verhütet und behandelt Ketose und verbessert die Futterverwertung in Wiederkäuern oder Schweinen. Der verantwortliche Mechanismus für Ketose ist eine verminderte Produktion von Propionatverbindungen. Eine gegenwärtig empfohlene Behandlung ist die Verabreichung von Propionsäure oder von Futter, welches vorzugsweise Propionate produziert. Es ist klar, dass das Anregen der Propionatproduktion von herkömmlichen Futter das Auftreten von Ketose reduziert.

Es wurde gefunden, dass die Aether von X-14868A die Effizienz der Futterverwertung in Wiederkäuern erhöhen, wenn diese Aether von X-14868A den Tieren oral verabreicht werden. Der einfachste Weg zur Verabreichung der Antibiotika besteht darin, diese in das Tierfutter einzumischen.

Die Antibiotika können jedoch auch in anderer Weise nutzvoll verabreicht werden. Sie können beispielsweise in Tabletten, Tränken, Boli oder Kapseln eingearbeitet werden und so den Tieren verabreicht werden. Formulierungen der antibiotischen Verbindungen in derartigen Dosierungsformen werden nach an sich in der Veterinärpharmazie bekannten Methoden hergestellt.

Kapseln werden in üblicher Weise hergestellt durch Auffüllen von Gelatinekapsel beliebiger Form mit den gewünschten Antibiotika. Erwünschtenfalls können die Antibiotika mit einem inerten, festen Verdünnungsmittel verdünnt werden, wie beispielsweise mit einem Zucker, Stärke oder gereinigter kristalliner Cellulose, um das Volumen aus Zweckmässigkeitsgründen zu erhöhen.

Tabletten werden durch herkömmliche pharmazeutische Methoden hergestellt. Eine Tablette enthält

im allgemeinen ausser dem Wirkstoff einen Grundstoff, einem Desintegrator, ein Absorbens, ein Bindemittel und ein Gleitmittel. Typische Grundstoffe sind z. B. Milchzucker, feiner Puderzucker, Natriumchlorid, Stärke und Mannit. Stärke ist ebenfalls ein guter Desintegrator, wie ebenso Alginsäure. Oberflächenaktive Mittel, wie Natriumlaurylsulfat und Dioctylnatriumsulfosuccinat werden gelegentlich ebenfalls verwendet. Ueblicherweise verwendete Absorbentien sind z. B. Stärke und Milchzucker, während Magnesiumcarbonat bei öligen Substanzen Verwendung findet. Oft verwendete Bindemittel sind Gelatine, Gummi arabicum, Stärke, Dextrin und verschiedene Cellulosederivate. Häufig verwendete Gleitmittel sind z. B. Magnesiumstearat, Talk, Paraffinwachs, verschiedene Metallseifen und Polyäthylenglykol.

Die Antibiotika können ebenfalls in Form von Boli mit verzögerter Wirkung verabreicht werden. Derartige Boli werden als Tabletten hergestellt, wobei jedoch Mittel zur Verzögerung der Auflösung der Antibiotika zugefügt werden. Boli sind für Freisetzung während längerer Perioden vorgesehen. Die langsame Auflösung wird durch Wahl einer stark wasserunlöslichen Form der Antibiotika gewährleistet. Beispielsweise können Eisenfeilenspäne zugegeben werden, um die Dichte des Bolos zu erhöhen, damit dieser am Boden des Pansens bleibt.

Die Auflösung der Antibiotika wird durch Verwendung einer Matrize unlöslichen Materials, in welcher der Wirkstoff eingebettet wird, verzögert. Beispielsweise können pflanzliche Wachse, gereinigte Mineralwachse und wasserunlösliche polymere Materialien verwendet werden.

Tränken der Antibiotika werden zweckmässig durch Wahl einer wasserlöslichen Form der Antibiotika hergestellt. Wird eine unlösliche Form gewünscht, kann eine Suspension hergestellt werden. Wahlweise kann eine Tränke als eine Lösung in einem physiologisch einwandfreien Lösungsmittel, wie beispielsweise Polyäthylenglykol, zubereitet werden.

Suspensionen von unlöslichen Formen der Antibiotika können in nicht-Lösungsmitteln hergestellt werden, wie beispielsweise in pflanzlichen Oelen, z. B. Erdnuss-, Mais- oder Sesamöl, in einem Glykol, wie z. B. Propylenglykol oder Polyäthylenglykol, oder in Wasser, je nach der gewählten Form des Antibiotikums.

Geeignete physiologisch verträgliche Zusätze sind notwendig, um die Antibiotika in Suspension zu halten. Die Zusätze können Verdickungsmittel sein, wie z. B. Carboxymethylcellulose, Polyvinylpyrrolidon, Gelatine oder Alginate. Verschiedene Klassen von Schaumerzeugern dienen dazu, das Antibiotikum in Suspension zu halten. Beispielsweise sind Lecithin, Alkylphenol/Polyäthylenoxidaddukte, Naphthalinsulfonate, Alkylbenzolsulfonate und Polyoxyäthylensorbitanester für die Herstellung von Suspensionen in flüssigen nicht-Lösungsmittel verwendbar.

Für die Herstellung von Suspensionen können zusätzlich verschiedene Substanzen, welche die Hydrophilität, Dichte und Oberflächenspannung beeinflussen, verwendet werden. Beispiele sind Silikonantischaummittel, Glykole, Sorbit und Zucker, die als Suspendierungsmittel Verwendung finden.

Die Antibiotika können dem Züchter als eine Suspension oder als trockenes Gemisch mit einem entsprechenden Hilfsmittel zur Verdünnung vor der Verwendung angeboten werden.

Die Antibiotika können ebenfalls dem Trinkwasser des Wiederkäuers zugeführt werden. Dabei wird eine wasserlösliche oder wassersuspendierbare Form der Antibiotika dem Trinkwasser in entsprechender Menge zugegeben. Die Zubereitung der Antibiotika für das Vermischen mit dem Trinkwasser folgt den gleichen Prinzipien wie die Formulierung von Tränken.

Die zweckmässigste Art, die Tiere mit dem Antibiotika zu behandeln, basiert auf der Verabreichung mit dem Tierfutter. Es können beliebige Tierfutter, z. B. übliche Trockenfutter, flüssige Futter und tablettierte Futter verwendet werden.

Die Methoden zur Einbringung der Arzneimittel in das Tierfutter sind bestens bekann. Ueblicherweise wird für ein medikiertes Futter eine konzentrierte Vormischung hergestellt. So z. B. können typische Arzneimittel enthaltende Futtermischungen von ungefähr 0,1 bis ungefähr 25 g des Arzneimittels pro Tonne Vormischung enthalten. Dieser weite Bereich ist durch den gewünschten, weiten Konzentrationsbereich für das fertige Futter bedingt. Vormischungen können sowohl in flüssigen als auch in fester Form vorliegen.

Die Formulierung von Wiederkäuerfutter enthaltend die genaue Menge an Antibiotika für eine übliche Behandlung ist bestens bekannt. Es ist nur notwendig, den Anteil einer Verbindung, welche für die Verabreichung jedes einzelnen Tieres erwünscht ist, zu berechnen und den Anteil an Futter pro Tag, welches das Tier frisst und die Konzentration der Antibiotika in der Vormischung zu berücksichtigen und dann die genaue Konzentration der Antibiotika oder der Vormischung im Futter zu berechnen.

Alle Methoden zur Formulierung, Mischung und Tablettierung von Futter, welche üblicherweise bei Wiederkäuernfutter verwendet werden, sind zur Herstellung von Futter enthaltend die antibiotischen Verbindungen geeignet.

Wie oben gezeigt, bewirkt die orale Verabreichung der Antibiotika eine vorteilhafte Aenderung der Erzeugung von Propionat im Verhältnis zu Acetat in Pansen. Es kann deshalb postuliert werden, dass die gleiche Behandlung ebenfalls monogastrische Tiere, die faseriges Pflanzenmaterial im Blinddarm vergären, günstig beeinflusst, denn es kann erwartet werden, dass eine vorteilhafte Veränderung in Propionat/Acetatverhältnis nach oraler Gabe der Antibiotika auftreten wird. Pferde, Schweine und Kaninchen sind Beispiele von Tieren, welche einen Teil ihres Futters zur Fermentation im Blinddarm verdauen.

Bestimmung der Produktion von flüchtigen Fettsäuren

Ein Rind, chirurgisch modifiziert durch eine Fistel am Pansen, wird als Quelle der Pansenflüssigkeit verwendet. Die Integrität des Pansens wird durch eine Pansenkanüle (Bar Diamond Labs, Parma, Idaho) gewährleistet, welche zum Zweck der Entnahme von Pansenflüssigkeitsproben geöffnet wird. Das Tier wird zweimal täglich mit einer Mischung von 68 % Konzentrat (AHRES-Ration = 39) zu 20 % Getreide gefüttert. Die Pasenflüssigkeit wird vor der morgigen Fütterung entnommen. Die Pansenflüssigkeit wird durch 4 Schichten Käsetuch in einen 1 Gallon Nalgene-Behälter gesiebt und unter Kohlendioxid aufbewahrt. 1 000 ml der gesiebten Pansenflüssigkeit werden zu 2 000 ml eines eiskalten Puffers gegeben, welcher durch Cheng et al. in J. Dair. Sci., 38, 1225 (1955) spezifiziert wird. Die Zusammensetzung dieses Puffers ist wie folgt:

| | | | |
|---|---|---|---|
| $Na_2HPO_4$ | 0,316 g/l | $MgSO_4$ | 0,112 |
| $KH_2PO_4$ | 0,152 | $CaCl_2$ | 0,038 |
| $NaHCO_3$ | 2,260 | $FeSO_4 \cdot 7H_2O$ | 0,008 |
| NaCl | 0,375 | $ZnSO_4 \cdot 7H_2O$ | 0,004 |
| KCl | 0,375 | $CuSO_4 \cdot 5H_2O$ | 0,002 |

Die gepufferte Pansenflüssigkeit wird in einem 1 l Scheidetrichter gehalten. Um den anaeroben Charakter und die Homogenität der gepufferten Pansenflüssigkeit zu erhalten, wird konstant $CO_2$ durch die Flüssigkeit gelassen. Für die individuellen Fermentationen werden 125 ml Erlenmeyer-Kolben verwendet. Jeder Kolben, der mit einer Verbindung versetzt werden soll, enthält 0,75 g von fein gemahlenem 80 % Konzentrat : 20 % Alfaalfa-Heu. Kolben, welche für die arzneimittelfreien Kontrollen verwendet werden, enthalten 0,82 g der fein gemahlenen Basisration. 0,6 ml der Testverbindung werden in einem geeigneten Lösungsmittel gelöst und zu jedem Kolben gegeben, worauf man über Nacht absitzen lässt. Jede Verbindung wird bei einer Endkonzentration von 50 ppm untersucht. Lösungsmittel ohne Testverbindung wird zu den arzneimittelfreien Kontrollfermentationskolben gegeben. Monensin bei 50 ppm wird als positive Kontrolle verwendet.

60 g gepufferte Pansenflüssigkeit werden zu jedem Kolben mit Testverbindung gegeben, und 65,9 g werden den Kontrollkolben zugegeben. Kolben, bei denen alle Komponenten zugegeben wurden, werden mit einem Verschluss verschlossen, welcher ein Einweggasventil aufweist, welches das Entweichen der Gase ermöglicht, die durch die Fermentation hergestellt wurden. 6 ml Proben werden allen Kontrollkolben entnommen (« 0-Zeit »-Proben). Die Kolben werden während 4 Stunden unter Schütteln (120 Bewegungen pro Minute) inkubiert.

Pansenflüssigkeit wird in 25 × 150 ml Glasröhrchen gegossen und während ungefähr 15 Minuten in einem Eisbad zur Ausscheidung von grober Materie stehen gelassen. 2 ml 25 % (Gewicht/Volumen) Metaphosphorsäure (J. T. Baker) in 13 ml Polycarbonatzentrifugenröhrchen (Autoclear, IEC) werden mit 6 ml Pansenflüssigkeit versetzt. Jedes Röhrchen wird verschlossen und gründlich gemischt. Die Röhrchen werden während 30 Minuten bei Raumtemperatur stehen gelassen und dann während 10 Minuten bei 16 000 upm in einer « 874 angle head » in einer IEC B20-Zentrifuge zentrifugiert. 1 ml interner Standard (0,25 % 2-Methylvaleriansäure, Aldrich Chemical Company) wird dann zu 4 ml Ueberstand gegeben. Die erhaltene Mischung wird unter Verwendung eines « Swinnex » Filter und einer 5 ml Spritze durch ein 0,22 Micron Milliporfilter filtriert. Das Filtrat wird in 1 ml Glasröhrchen mit gepresster Gummimembran versiegelt.

Jedes Röhrchen, das je eine individuelle Fermentation repräsentiert, wird durch Gasflüssigchromatographie auf flüchtige Fettsäuren analysiert.

Jedes Röhrchen wird durch drei aufeinanderfolgende Injektion an Kolonnen analysiert. Die Konzentrationen von Acetat, Propionat, i-Butyrat, n-Butyrat, i-Valerat und n-Valerat werden durch Vergleich mit Analysen einer Standardlösung von flüchtigen Fettsäuren unter Verwendung einer internen Standardisierungsmethode berechnet.

Die Aktivität einer Verbindung wird durch Messen der relativen Aenderungen in der Produktion von Propionat ($C_3$) und Acetat ($C_2$) und n-Butyrat ($C_4$) bestimmt, d. h. durch das molare Verhältnis der flüchtigen Fettsäuren $C_3/C_2 + C_4$. Die Zunahme im Verhältnis ($C_3/C_2 + C_4$) welches mit jeder Verbindung gegenüber der Kontrolle erhalten wird, wird als Prozentsatz der Zunahme ausgedrückt, welches mit der positiven Kontrolle erhalten wird. Die Resultate sind in der nachfolgenden Tabelle angegeben.

(Siehe Tabelle Seite 7 f.)

Molares Verhältnis von flüchtigen Fettsäuren hergestellt in Fermentationen von Verbindungen bei 50 ppm

| Verbindung | molares Verhältnis der flüchtigen Fettsäuren $C_3/C_2 + C_4$ | Zunahme gegenüber Kontrolle | % der Zunahme mit Monensin |
|---|---|---|---|
| Unbehandelte Kontrolle | 0.356 | - | - |
| Monensin | 0.549 | 0.193 | 100 |
| n-Propyläther von X-14868A | 0.497 | 0.141 | 73.1 |
| Aethyläther von X-14868A | 0.541 | 0.185 | 95.9 |
| n-Butyläther von X-14868A | 0.502 | 0.146 | 75.6 |
| Aethylenglycoläther von X-14868A | 0.574 | 0.218 | 113.0 |
| n-Hexyläther von X-14868A | 0.549 | 0.193 | 100.0 |
| n-Pentyläther von X-14868A | 0.482 | 0.126 | 65.3 |
| Butylenglycoläther von X-14868A | | 0.174 | 99.4 |

Die Salze der Säureform der Aether werden nach Methoden, die bei Polyätherverbindungen bestens bekannt sind, hergestellt, z. B. durch Waschen der freien Säuren in einer Lösung mit einer geeigneten Base oder einem Salz. Beispiele von solchen pharmazeutisch annehmbaren Basen, welche fähig sind, für die Zwecke der vorliegenden Erfindung Salze zu liefern, umfassen Alkalimetallbasen, wie Natriumhydroxid, Kaliumhydroxid, Lithiumhydroxid und dergleichen; Erdalkalimetallbasen, wie Calciumhydroxid, Bariumhydroxid und dergleichen und Ammoniumhydroxid. Alkalimetall- oder Erdalkalimetallsalze, welche zur Bildung geeigneter pharmazeutisch annehmbarer Säureadditionssalze geeignet sind, umfassen Anionen, wie Carbonate, Bicarbonate und Sulfate.

Beispiele organischer Basen, welche mit den Polyätherverbindungen pharmazeutisch annehmbare Salze bilden, sind niedere Alkylamine und primäre, sekundäre und tertiäre Hydroxy niedere Alkylamine wie z. B. Aethylamin, Isopropylamin, Diäthylamin, Methyl-n-butylamin, Aethanolamin und Diäthanolamin.

Ein speziell bevorzugtes Amin ist N-Methylglucamin. Salze von N-Methylglucamin sind wegen ihrer Wasserlöslichkeit, welche sie für die parenterale Verwendung geeignet machen, von speziellem Wert.

Die Verbindungen der vorliegenden Erfindung sind typischerweise aktiv beim Erhöhen der Effizienz der Futterverwertung wenn sie Wiederkäuern oral in Dosen von ungefähr 0,003 mg/kg/Tag bis ungefähr 0,75 mg/kg/Tag verabreicht werden. Bestbefriedigende Ergebnisse erhält man bei Dosen von ungefähr 0,03 mg/kg/Tag bis ungefähr 0,075 mg/kg/Tag.

## Beispiel 1

Herstellung des Aethyläthers des Antibiotikums X-14868A als freie Säure

Die freie Säure des Antibiotikums X-14868A (1,2 g, 0,001 28 Mol) wird in absolutem Aethanol (40 ml) gelöst, worauf Ionenaustauscherharz AG 50W-X4 (200-400 Mesh) in der $H^+$-Form (2,0 g über Nacht unter Aethanol gelagert, filtriert und vor Gebrauch mit frischem Aethanol gewaschen) zugegeben. Dieses Gemisch wird während 3 Stunden bei Raumtemperatur gerührt, worauf man das Harz abtrennt und mit Aethanol wäscht. Die vereinigten Aethanolfiltrate werden zu einem Volumen von ungefähr 10 ml konzentriert und diese Lösung wird über Nacht in der Kälte (0-5 °C) gehalten.

Durch Filtration erhält man das gewünschte Produkt als weisse Kristalle vom Schmelzpunkt 166-168 °C.

Beispiel 2

Herstellung des n-Propyläthers des Antibiotikums X-14868A als freie Säure

Die freie Säure des Antibiotikums X-14868A (6,2 g, 0,006 6 Mol) wird bei Raumtemperatur in 1-Propanol (80 ml) gelöst, und es wird Ionenaustauschharz AG 50W-X4 (200-400 Mesh) in der $H^+$-Form (6,0 g über Nacht unter 1-Propanol gelagert, filtriert und vor Gebrauch mit 1-Propanol gewaschen) zugegeben. Das erhaltene Gemisch wird entweder 4 Stunden bei Raumtemperatur gerührt oder solange gerührt, bis Dünnschichtchromatographie (Aether, Hexan, Methanol, Aceton $NH_4OH$ in einem Verhältnis von 7 : 3 : 0,5 : 1 : 0,02) zeigt, dass die Reaktion beendet ist.

Man filtriert das Harz ab und wäscht mit 1-Propanol. Die vereinigten Filtrate werden zuerst am Rotationsverdampfer konzentriert und dann am Wasserstrahlvakuum und geben das gewünschte Produkt in Form eines weissen Schaums. Eine Probe, die man aus einer minimalen Menge von 1-Propanol kristallisiert, zeigt den Schmelzpunkt 109-111 °C (Zers.).

Beispiel 3

Herstelung des n-Butyläthers des Antibiotikums X-14868A als Natriumsalz

1 g des Natriumsalzes des Antibiotikums X-14868A wird bei Raumtemperatur unter Rühren in 20 ml n-Butylalkohol gelöst. Zu dieser Lösung gibt man 5 g AG50W-X4 (200-400 Mesh) in der $H^+$-Form, welches Harz vorgängig über Nacht in n-Butylalkohol eingeweicht wurde, dann filtriert und mit n-Butylalkohol gewaschen wurde. Das Reaktionsgemisch wird während 4 Stunden bei Raumtemperatur gerührt.

Das Reaktionsgemisch wird filtriert und das Filtrat wird im Vakuum konzentriert. Der Rückstand wird in Aethylacetat gelöst und mit Natriumcarbonatlösung (bei Raumtemperatur gesättigt) gewaschen, dann mit Wasser gewaschen und über Natriumsulfat getrocknet. Nach Filtration und Konzentration kristallisiert man den Rückstand aus Acetonitril durch die Zugabe von Wasser. Schmelzpunkt 146-150 °C.

Beispiel 4

Herstellung des n-Butyläthers des Antibiotikums X-14868A als Natriumsalz

1 g Natriumsalz des Antibiotikums X-14868A wird unter Rühren bei Raumtemperatur in 20 ml n-Butylalkohol gelöst. Zu dieser Lösung gibt man 5 g AG50W-X4 (200-400 Mesh) in der $H^+$-Form, welches Harz vorgängig über Nacht in n-Butylalkohol eingeweicht, filtriert und mit n-Butylalkohol gewaschen wurde. Das Reaktionsgemisch wird über Nacht bei Raumtemperatur gerührt. Das Reaktionsgemisch wird filtriert und im Vakuum konzentriert. Der Rückstand wird in Methylenchlorid gelöst und mit Natriumcarbonat (gesättigt bei Raumtemperatur) gewaschen und anschliessend mit Wasser gewaschen und dann über Natriumsulfat getrocknet. Nach Filtration wird die Lösung konzentriert und an einer 200 g mit Methylenchlorid präparierten Silicagelsäure chromatographiert, wobei mit einem Liter Methylenchlorid, 3 l Diäthyläther/Hexan/Aceton (7 : 3 : 1), gefolgt von 2 l Diäthyläther/Aceton (7 : 3) eluiert wird. Fraktionen die das Reaktionsprodukt enthalten werden konzentriert und durch Zugabe von Wasser aus Acetonitril kristallisiert. Schmelzpunkt 155 °C.

Beispiel 5

Herstellung des n-Amyläthers des Antibiotikums X-14868A als Natriumsalz

Der n-Amyläther des Antibiotikums X-14868A als Natriumsalz wird wie in Beispiel 3 beschrieben hergestellt, wobei jedoch n-Amylalkohol an Stelle von n-Butylalkohol verwendet wird. Schmelzpunkt 136-140 °C.

Beispiel 6

Herstellung des n-Hexyläthers des Antibiotikums X-14868A als Natriumsalz

Der n-Hexyläther des Antibiotikums X-14868A als Natriumsalz wird wie in Beispiel 3 beschrieben hergestellt, wobei jedoch n-Hexylalkohol an Stelle von n-Butylalkohol verwendet wird. Schmelzpunkt 137-139 °C.

Beispiel 7

Herstellung des Aethylenglykoläthers des Antibiotikums X-14868A als Natriumsalz

6 g Natriumsalz X-14868A werden in einem minimalen Volumen von Methylenchlorid/Aceton gelöst.

Nach Zugabe von Aethylenglykol (100 ml) und 30 g AG50W-X4 (200-400 Mesh) Harzes in der $H^+$-Form (das Harz wird vorgängig über Nacht in Aethylenglykol eingeweicht) wird das Reaktionsgemisch über Nacht bei Raumtemperatur gerührt. Zum Reaktionsgemisch wird Wasser gegeben, das Harz wird abfiltriert und das Filtrat wird dreimal mit Diäthyläther extrahiert. Die Aetherphasen werden mit Wasser, Natriumcarbonat, Wasser und dann wieder mit Wasser gewaschen und über Natriumsulfat getrocknet. Die flüssige Phase wird im Vakuum entfernt, und der Rückstand wird aus Acetonitril durch Zugabe von Wasser kristallisiert. Schmelzpunkt 162-165 °C.

Beispiel 8

Herstellung des Aethylenglycoläther des Antibiotikums X-14868A als Kaliumsalz

Der Aethylenglycyläther des Antibiotikums X-14868A als Kaliumsalz wird wie in Beipsiel 7 beschrieben hergestellt, doch wird anstelle von Natriumcarbonat Kaliumhydroxid verwendet und das Trocknen der ätherischen Extrakte des Reaktionsgemisches erfolgt durch Filtrieren durch Celit. Schmelzpunkt 158-162 °C.

Beispiel 9

Herstellung des 1,4-Butylenglycoläthers des Antibiotikums X-14868A als Natriumsalz

Der 1,4-Butylenglycyläther des Antibiotikums X-14868A als Natriumsalz wird wie in Beipsiel 7 beschrieben hergestellt, ausgenommen dass 1,4-Butandiol anstelle von Aethylenglykol verwendet wird. Das Produkt ist ein weisser (amorpher) Schaum $[\alpha]_D^{25} = 22,7°$ in Chloroform, c = 1 %.

Beispiel 10

2-Propanoläther des Antibiotikums X-14868A als freie Säure

Das Natriumsalz des ionophoren Antibiotikums X-14868A (1,1 g) wird in 2-Propanol (50 ml) bei Raumtemperatur gelöst, worauf man Ionenaustauschharz AG50W-X4 (200-400 Mesh) in der $H^+$-Form (1,1 g gelagert unter 2-Propanol, filtriert und mit 2-Propanol vor der Verwendung gewaschen) zugibt. Das Gemisch wird während 24 Stunden bei raumtemperatur gerührt. Das Harz wird filtriert und mit frischem Isopropylalkohol gewaschen. Das Filtrat wird zuerst im Rotationsverdampfer filtriert und dann an der Wasserstrahlpumpe und liefert einen weissen Schaum. Mikroanalyse: Berechnet für $C_{50}H_{86}O_{17}$: C, 62,61 ; H, 9,09
C, 62,73 ; H, 8,81.

Beispiel 11

Herstellung des 1,3-Propandiol (Trimethylenglykol)äther des Antibiotikums X-14868A als Natriumsalz

5 g Natriumsalz des Antibiotikums X-14868A wird in 100 ml 1,3-Propandiol (Trimethylenglycol) unter Rühren bei Raumtemperatur gelöst. Zu dieser Lösung gibt man 20 g AG502-X8 (200-400 Mesh) Harz in der $H^+$-Form, wobei das Harz vorgängig über Nacht in Trimethylenglykol eingeweicht, filtriert und mit Trimethylenglykol gewaschen wurde. Das Reaktionsgemisch wird während 3 Stunden bei Raumtemperatur gerührt. Nach Zugabe von Wasser zum Reaktionsgemisch wird das Harz abfiltriert und das Filtrat zweimal mit Diäthyläther extrahiert. Die vereinigten Aetherphasen werden mit Wasser, Natriumcarbonat und wieder mit Wasser gewaschen und über Natriumsulfat getrocknet. Die Lösungsmittelphase wird zu einem Oel chromatographiert und an einer mit Methylenchlorid präparierten Silicagelsäule (200 g) chromatographiert, wobei man mit 1 l Diäthyläther/Hexan (7 : 3) 2 l Diäthyläther/Hexan/Aceton (7 : 3 : 1) 1 l Aethylacetat/Aceton (8 : 2) gefolgt durch 1 l Aethylacetat/Aceton (6 : 4) eluiert. Die Fraktionen welche das Reaktionsprodukt enthalten werden vereinigt. Durch Entfernung des Lösungsmittels im Vakuum erhält man einen weissen Schaum. Mikroanalyse : Berechnet für $C_{50}H_{85}NaO_{18} \cdot 1/2H_2O$ (1006.23) Berechnet für C 59,68, H 8,62, Na 2,29, $H_2O$ 0,90. Gefunden C 59,91, H 8,76, Na 2,08 $H_2O$ 1,02 ; $[\alpha]_D^{25}$ 24,3° in Chloroform, c = 1 %.

Beispiel 12

Herstellung von (S) und (R) 3-Hydroxybutyläther des Antibiotikums X-14868A als Natriumsalz

Der (S) und (R) 3-Hydroxybutyläther des Antibiotikums X-14868A als Natriumsalz wird wie in Beispiel 11 beschrieben hergestellt, doch werden 10 g Natriumsalz X-14868A verwendet und 1,3 Butandiol anstelle von Trimethylenglycol verwendet. Die Fraktionen 106-113 der Silicagelchromatographie liefern einen weissen Schaum des (S) 3-Hydroxybutyläther von X-14868A als Natriumsalz. Mikroanalyse : Berechnet für

$C_{51}H_{87}NaO_{18}$ (1011.25). Berechnet C 60,58 ; H 8,67 ; Na 2,27. Gefunden : C 60,52 ; H 8,62 ; Na 2,29 ; $[\alpha]_D^{25}$ 28,3° in Chloroform, c = 1 %. Die Fraktionen 126-170 der Silicagelchromatographie liefern einen weissen Schaum, nämlich (R) 3-Hydroxybutyläther des Antibiotikums X-14868A als Natriumsalz. Mikroanalyse : Berechnet für $C_{51}H_{87}NaO_{18}$ (1011.25). Berechnet C 60,58 ; H 8,67 ; Na 2,27. Gefunden : C 60,08 ; H 8,99 ; Na 2,07 ; $[\alpha]_D^{25}$ 25,9° in Chloroform, c = 1 %.

Die Identität des (S) und (R) 3-Hydroxybutyläthers des Antibiotikum X-14868A als Natriumsalz wurden unter Verwendung von 80 % reinem (S) und (R)-1,3-Butandiol für die Reaktion und vergleichende Dünnschichtchromatographie bestätigt.

## Beispiel 13

Herstellung des 5-Hydroxypentyläthers des Antibiotikums X-14868A als Natriumsalz

Der 5-Hydroxypentyläther des Antibiotikums X-14868A als Natriumsalz wird wie in Beispiel 11 beschrieben hergestellt, doch werden 10 g Natriumsalz des Antibiotikums X-14868A und 1,5-Pentandiol anstelle von Trimethylenglykol verwendet. Man erhält einen weissen Schaum, nämlich den 5-Hydroxypentyläther des Antibiotikums X-14868A als Natriumsalz. Mikroanalyse : Berechnet für $C_{52}H_{89}NaO_{18}$ (1025,27) Berechnet C 60,92 ; H 8,75 ; Na 2,24. Gefunden : C 60,78 ; H 8,95 ; Na 2,14 ; $[\alpha]_D^{25}$ 25,7° in Chloroform, c = 1 %.

## Beispiel 14

Herstellung des 1,2-Propandioläthers des Antibiotikums X-14868A als Natriumsalz

Gemäss Verfahren von Beispiel 11 jedoch unter Verwendung von 1,2-Propandiol anstelle von 1,3-Propandiol erhält man den 1,2-Propandioläther des Antibiotikums X-14868A.

**Patentansprüche** (für die Vertragsstaaten : BE, CH, DE, FR, GB, IT, LI, LU, NL, SE)

1. Eine Verbindung der Formel

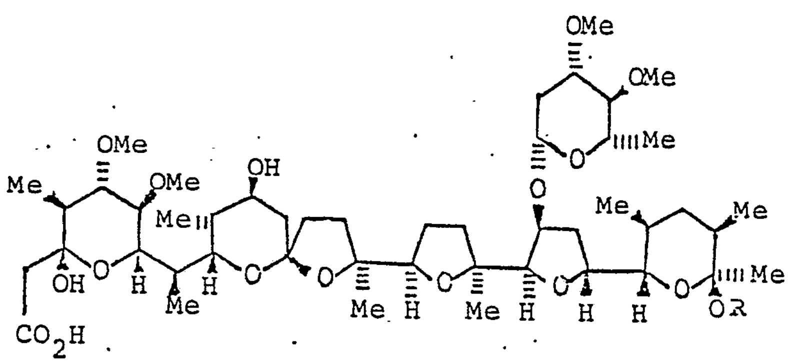

worin R $C_2$- bis $C_7$-Alkyl oder $C_2$-$C_7$-Hydroxyalkyl ist, sowie pharmazeutisch annehmbare Salze davon.

2. Eine Verbindung gemäss Anspruch 1, worin R Aethyl, n-Propyl, Isopropyl, n-Butyl, n-Pentyl, n-Hexyl, 1,2-Dihydroxyäthyl oder 1,4-Dihydroxybutyl ist und pharmazeutisch annehmbare Salze davon.

3. Eine Verbindung gemäss Anspruch 1, worin R 1,3 oder 1,2-Dihydroxypropyl, (S) oder (R) 3-Hydroxybutyl oder 5-Hydroxypentyl bedeutet, und pharmazeutisch annehmbare Salze davon.

4. Der Aethyläther des Antibiotikums X-14868A und seine pharmazeutisch annehmbaren Salze.

5. Der Aethylenglycoläther des Antibiotikums X-14868A und seine pharmazeutisch annehmbaren Salze.

6. Der n-Hexyläther des Antibiotikums X-14868A und seine pharmazeutisch annehmbaren Salze.

7. Der 1,4-Butylenglycoläther des Antibiotikums X-14868A und seine pharmazeutisch annehmbaren Salze.

8. Der 1,3-Propandioläther des Antibiotikums X-14868A und seine pharmazeutisch annehmbaren Salze.

9. Der (S)-3-Hydroxybutyläther des Antibiotikums X-14868A und seine pharmazeutisch annehmbaren Salze.

10. Der (R)-3-Hydroxybutyläther des Antibiotikums X-14868A und seine pharmazeutisch annehmbaren Salze.

11. Der 1,2-Propandioläther des Antibiotikums X-14868A und seine pharmazeutisch annehmbaren Salze.

12. Eine Verbindung der Formel

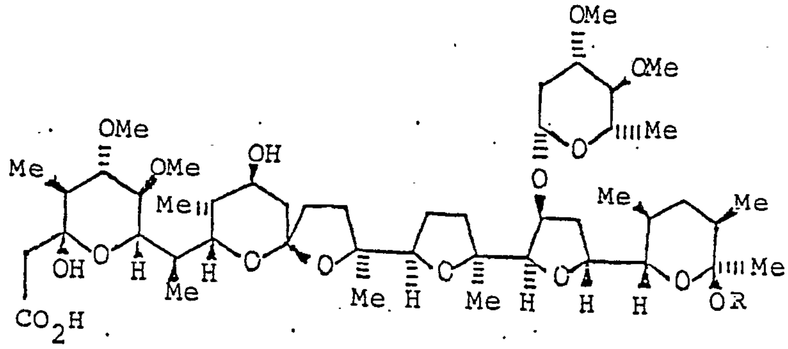

worin R $C_2$-$C_7$-Alkyl oder $C_2$-$C_7$-Hydroxyalkyl bedeutet und pharmazeutisch annehmbare Salze davon als Antibiotikum.

13. Eine Verbindung gemäss Anspruch 12 oder ein pharmazeutisch annehmbares Salz davon als anticoccidiostatisches Mittel.

14. Eine Verbindung gemäss Anspruch 12 oder ein pharmazeutisch annehmbares Salz davon als Antimalariamittel.

15. Eine Verbindung gemäss Anspruch 12 oder ein pharmazeutisch annehmbares Salz davon als ein Mittel zur Verbesserung der Futterverwertung in Wiederkäuern und Schweinen.

16. Verfahren zur Herstellung einer Verbindung der Formel

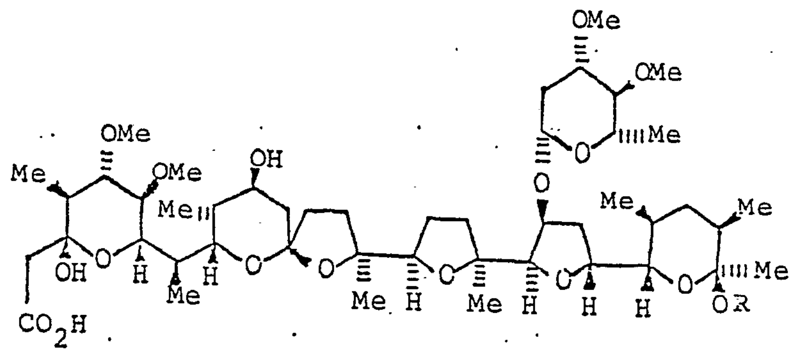

worin R $C_1$-$C_7$ Alkyl oder $C_2$-$C_7$ Hydroxyalkyl bedeutet, dadurch gekennzeichnet, dass man eine Verbindung der Formel

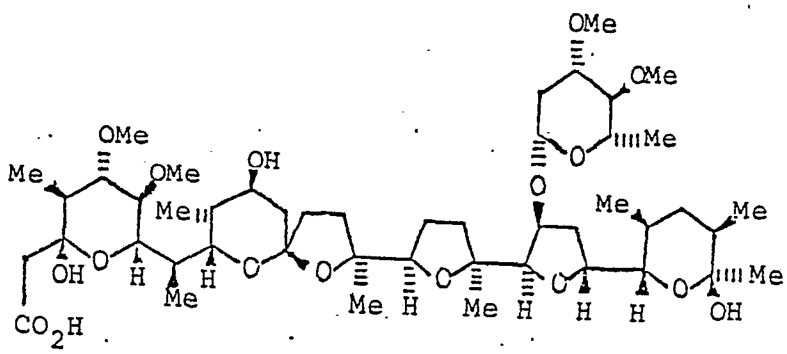

mit einem primären oder sekundären $C_1$-$C_7$-Alkohol oder einem $C_2$-$C_7$ difunktionellen Alkohol in Gegenwart eines sauren Ionenaustauscherharzes in der $H^+$-Form umsetzt.

17. Antibiotikum, dadurch gekennzeichnet, dass es eine Verbindung der Formel

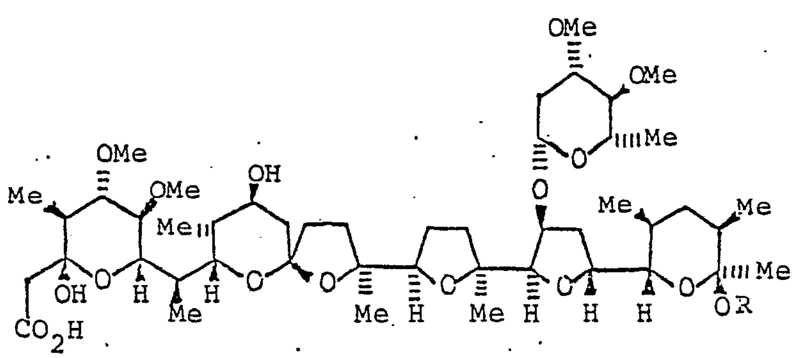

worin R $C_2$-$C_7$ Alkyl oder $C_2$-$C_7$ Hydroxyalkyl bedeutet, oder ein pharmazeutisch annehmbares Salz davon, sowie Trägermaterial enthält.

18. Ein anticoccidiostatisches Mittel, dadurch gekennzeichnet, dass es eine Verbindung der in Anspruch 17 angegebenen Formel oder ein pharmazeutisch annehmbares Salz davon, sowie Trägermaterial enthält.

19. Ein Antimalariamittel, dadurch gekennzeichnet, dass es eine Verbindung der in Anspruch 17 angegebenen Formel oder ein pharmazeutisch annehmbares Salz davon, sowie Trägermaterial enthält.

20. Ein Mittel zur Verbesserung der Futterverwertung in Wiederkäuern oder Schweinen, dadurch gekennzeichnet, dass es eine Verbindung der in Anspruch 17 angegebenen Formel oder ein pharmazeutisch annehmbares Salz davon, sowie Trägermaterial enthält.

21. Die Verwendung einer Verbindung gemäss einem der Ansprüche 1 bis 11 zur Herstellung eines Antibiotikums.

22. Die Verwendung einer Verbindung gemäss einem der Ansprüche 1 bis 11 zur Herstellung eines anticoccidiostatischen Mittels.

23. Die Verwendung einer Verbindung gemäss einem der Ansprüche 1 bis 11 zur Herstellung eines Antimalariamittels.

24. Die Verwendung einer Verbindung gemäss einem der Ansprüche 1 bis 11 als Mittel zur Verbesserung der Futterverwertung in Wiederkäuern und Schweinen.

**Patentansprüche** (für den Vertragsstaat AT)

1. Verfahren zur Herstellung einer Verbindung der Formel

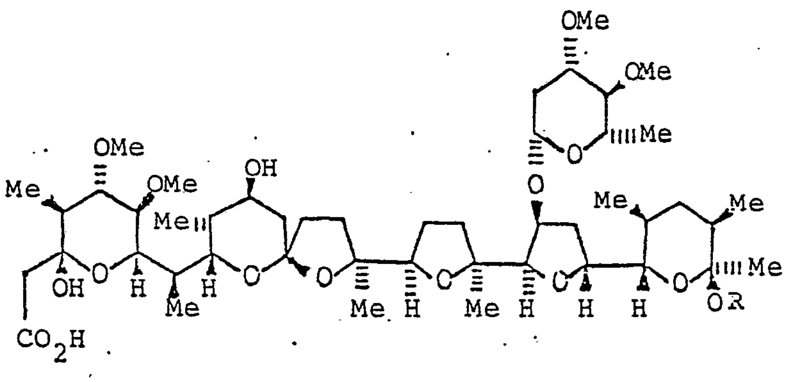

worin R $C_1$-$C_7$ Alkyl oder $C_2$-$C_7$ Hydroxyalkyl bedeutet, dadurch gekennzeichnet, dass man eine Verbindung der Formel

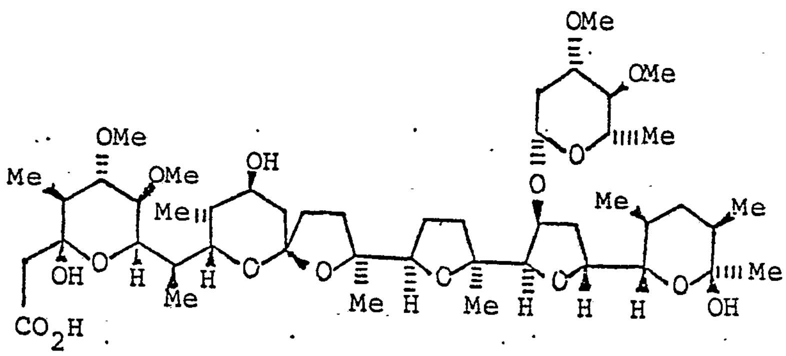

mit einem primären oder sekundären $C_1$-$C_7$-Alkohol oder einem $C_2$-$C_7$ difunktionellen Alkohol in Gegenwart eines sauren Ionenaustauscherharzes in der $H^+$-Form umsetzt.

2. Verfahren gemäss Anspruch 1, dadurch gekennzeichnet, dass man eine Verbindung der Formel I oder ein pharmazeutisch annehmbares Salz davon herstellt, worin R Aethyl, n-Propyl, Isopropyl, n-Butyl, n-Pentyl, n-Hexyl, 1,2-Dihydroxyäthyl oder 1,4-Dihydroxybutyl bedeutet.

3. Verfahren nach Anspruch 1, dadurch gekennzeichnet, dass man eine Verbindung der Formel I oder ein pharmazeutisch annehmbares Salz davon herstellt, worin R 1,3- oder 1,2-Dihydroxypropyl, (S) oder (R) 3-Hydroxybutyl oder 5-Hydroxypentyl bedeutet.

4. Verfahren nach Anspruch 1, dadurch gekennzeichnet, dass man den Aethyläther des Antibiotikums X-14868A oder ein pharmazeutisch annehmbares Salz davon herstellt.

5. Verfahren nach Anspruch 1, dadurch gekennzeichnet, dass man den Aethylenglycoläther des Antibiotikums X-14868A oder ein pharmazeutisch annehmbares Salz davon herstellt.

6. Verfahren nach Anspruch 1, dadurch gekennzeichnet, dass man den n-Hexyläther des Antibiotikums X-14868A oder ein pharmazeutisch annehmbares Salz davon herstellt.

7. Verfahren nach Anspruch 1, dadurch gekennzeichnet, dass man den 1,4-Butylenglycoläther des Antibiotikums X-14868A oder ein pharmazeutisch annehmbares Salz davon herstellt.

8. Verfahren nach Anspruch 1, dadurch gekennzeichnet, dass man den 1,3-Propandioläthers des Antibiotikums X-14868A oder ein pharmazeutisch annehmbares Salz davon herstellt.

9. Verfahren nach Anspruch 1, dadurch gekennzeichnet, dass man den (S)-3-Hydroxybutyläther des Antibiotikums X-14868A oder ein pharmazeutisch annehmbares Salz davon herstellt.

10. Verfahren nach Anspruch 1, dadurch gekennzeichnet, dass man den (R)-3-Hydroxybutyläther des Antibiotikums X-14868A oder ein pharmazeutisch annehmbares Salz davon herstellt.

11. Verfahren nach Anspruch 1, dadurch gekennzeichnet, dass man den 1,2-Propandioläther des Antibiotikums X-14868A oder ein pharmazeutisch annehmbares Salz davon herstellt.

**Claims** (for the Contracting States : BE, CH, DE, FR, GB, IT, LI, LU, NL, SE)

1. A compound of the formula

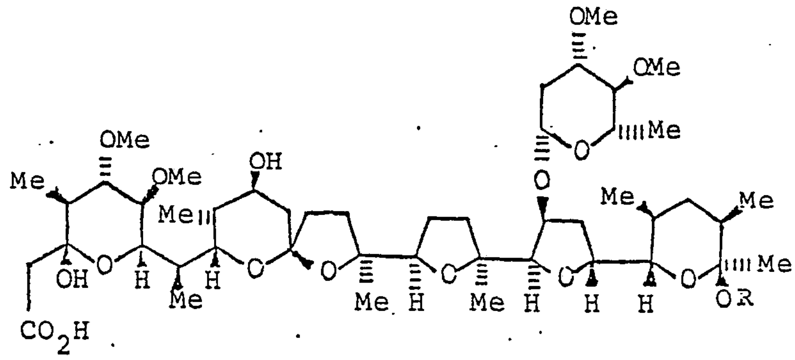

wherein R is $C_2$- to $C_7$-alkyl or $C_2$-$C_7$-hydroxyalkyl, as well as pharmaceutically acceptable salts thereof.

2. A compound in accordance with claim 1, wherein R is ethyl, n-propyl, isopropyl, n-butyl, n-pentyl, n-hexyl, 1,2-dihydroxyethyl or 1,4-dihydroxybutyl, and pharmaceutically acceptable salts thereof.

3. A compound in accordance with claim 1, wherein R signifies 1,3- or 1,2-dihydroxypropyl, (S) or (R) 3-hydroxybutyl or 5-hydroxypentyl, and pharmaceutically acceptable salts thereof.

4. The ethyl ether of antibiotic X-14868A and its pharmaceutically acceptable salts.

5. The ethylene glycol ether of antibiotic X-14868A and its pharmaceutically acceptable salts.

6. The n-hexyl ether of antibiotic X-14868A and its pharmaceutically acceptable salts.

7. The 1,4-butylene glycol ether of antibiotic X-14868A and its pharmaceutically acceptable salts.

8. The 1,3-propanediol ether of antibiotic X-14868A and its pharmaceutically acceptable salts.

9. The (S)-3-hydroxybutyl ether of antibiotic X-14868A and its pharmaceutically acceptable salts.

10. The (R)-3-hydroxybutyl ether of antibiotic X-14868A and its pharmaceutically acceptable salts.

11. The 1,2-propanediol ether of antibiotic X-14868A and its pharmaceutically acceptable salts.

12. A compound of the formula

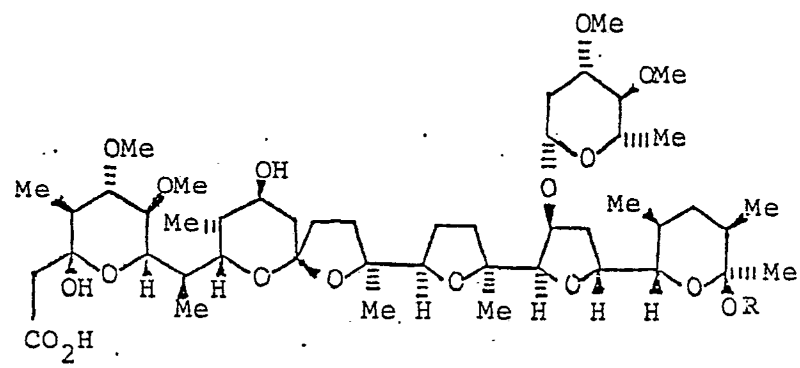

wherein R signifies $C_2$-$C_7$-alkyl or $C_2$-$C_7$-hydroxyalkyl, and pharmaceutically acceptable salts thereof as an antibiotic.

13. A compound in accordance with claim 12 or a pharmaceutically acceptable salt thereof as an anti-coccidiostatic agent.

14. A compound in accordance with claim 12 or a pharmaceutically acceptable salt thereof as an antimalarial agent.

15. A compound in accordance with claim 12 or a pharmaceutically acceptable salt thereof as an agent for improving feed utilization in ruminants and swine.

16. A process for the manufacture of a compound of the formula

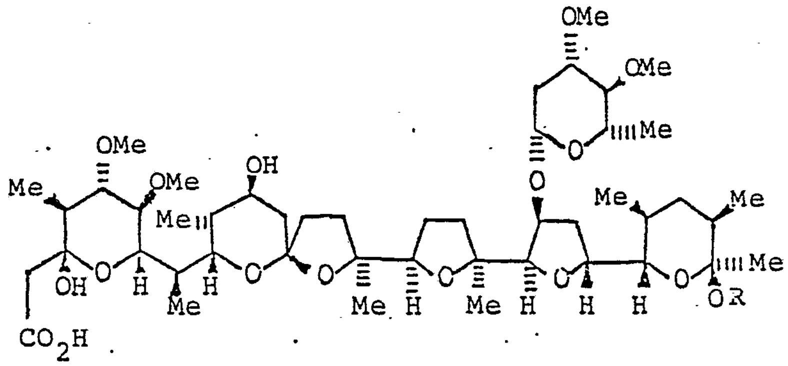

wherein R signifies $C_1$-$C_7$ alkyl or $C_2$-$C_7$ hydroxyalkyl, characterized by reacting a compound of the formula

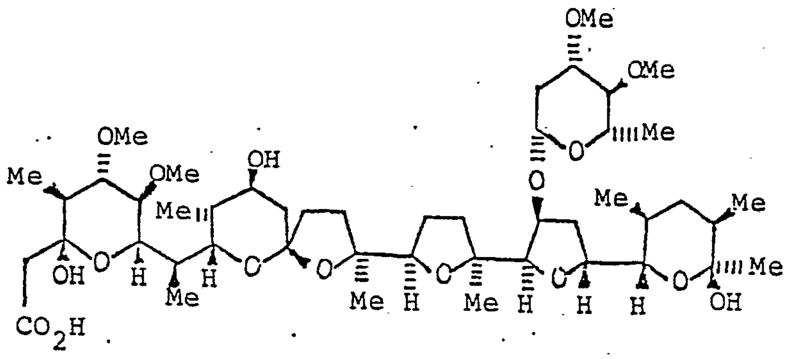

with a primary or secondary $C_1$-$C_7$-alcohol or a $C_2$-$C_7$ difunctional alcohol in the presence of an acidic ion exchange resin in the $H^+$ form.

17. An antibiotic, characterized in that it contains a compound of the formula

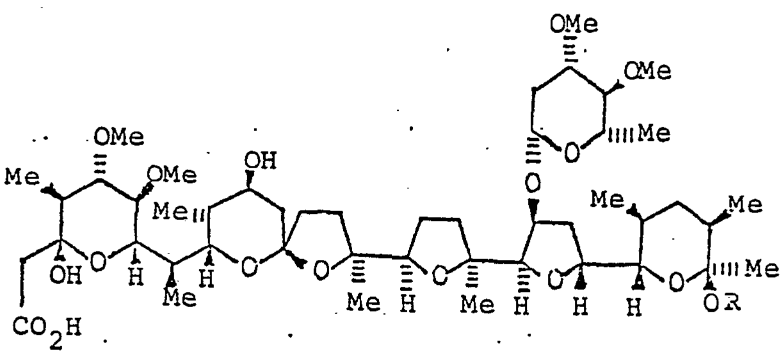

wherein R signifies $C_1$-$C_7$ alkyl or $C_2$-$C_7$ hydroxyalkyl, or a pharmaceutically acceptable salt thereof, as well as carrier material.

18. An anticoccidiostatic agent, characterized in that it contains a compound of the formula given in claim 17 or a pharmaceutically acceptable salt thereof, as well as carrier material.

19. An antimalarial agent, characterized in that it contains a compound of the formula given in claim 17 or a pharmaceutically acceptable salt thereof, as well as carrier material.

20. An agent for improving feed utilization in ruminants or swine, characterized in that it contains a compound of the formula given in claim 17 or a pharmaceutically acceptable salt thereof, as well as carrier material.

21. The use of a compound in accordance with any one of claims 1 to 11 for the manufacture of an antibiotic.

22. The use of a compound in accordance with any one of claims 1 to 11 for the manufacture of an anticoccidiostatic agent.

23. The use of a compound in accordance with any one of claims 1 to 11 for the manufacture of an antimalarial agent.

24. The use of a compound in accordance with any one of claims 1 to 11 as an agent for improving feed utilization in ruminants and swine.

**Claims** (for the Contracting State AT)

1. A process for the manufacture of a compound of the formula

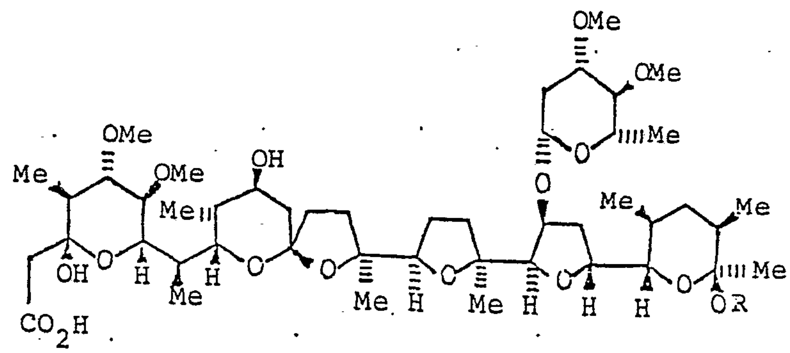

wherein R signifies $C_1$-$C_7$ alkyl or $C_2$-$C_7$ hydroxyalkyl, characterized by reacting a compound of the formula

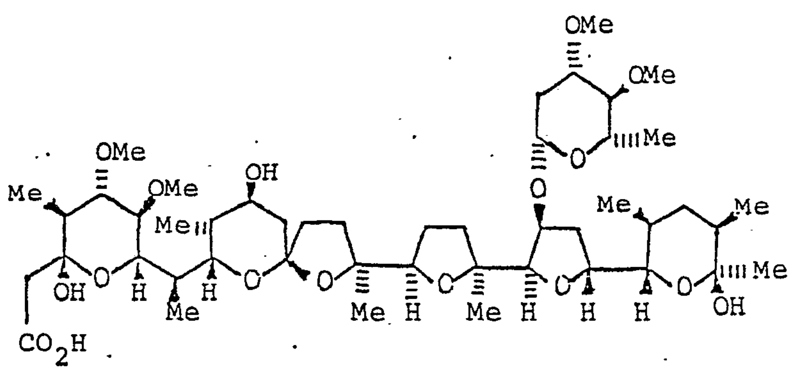

with a primary or secondary $C_1$-$C_7$-alcohol or a $C_2$-$C_7$ difunctional alcohol in the presence of an acidic ion exchange resin in the $H^+$ form.

2. A process in accordance with claim 1, characterized in that a compound of formula I or a pharmaceutically acceptable salt thereof, wherein R signifies ethyl, n-propyl, isopropyl, n-butyl, n-pentyl, n-hexyl, 1,2-dihydroxyethyl or 1,4-dihydroxybutyl, is manufactured.

3. A process according to claim 1, characterized in that a compound of formula I or a pharmaceutically acceptable salt thereof, wherein R signifies 1,3- or 1,2-dihydroxypropyl, (S) or (R) 3-hydroxybutyl or 5-hydroxypentyl, is manufactured.

4. A process according to claim 1, characterized in that the ethyl ether of antibiotic X-14868A or a pharmaceutically acceptable salt thereof is manufactured.

5. A process according to claim 1, characterized in that the ethylene glycol ether of antibiotic X-14868A or a pharmaceutically acceptable salt thereof is manufactured.

6. A process according to claim 1, characterized in that the n-hexyl ether of antibiotic X-14868A or a pharmaceutically acceptable salt thereof is manufactured.

7. A process according to claim 1, characterized in that the 1,4-butylene glycol ether of antibiotic X-14868A or a pharmaceutically acceptable salt thereof is manufactured.

8. A process according to claim 1, characterized in that the 1,3-propanediol ether of antibiotic X-14868A or a pharmaceutically acceptable salt thereof is manufactured.

9. A process according to claim 1, characterized in that the (S)-3-hydroxybutyl ether of antibiotic X-14868A or a pharmaceutically acceptable salt thereof is manufactured.

10. A process according to claim 1, characterized in that the (R)-3-hydroxybutyl ether of antibiotic X-14868A or a pharmaceutically acceptable salt thereof is manufactured.

11. A process according to claim 1, characterized in that the 1,2-propanediol ether of antibiotic X-14868A or a pharmaceutically acceptable salt thereof is manufactured.

**Revendications** (pour les états contractants : BE, CH, DE, FR, GB, IT, LI, LU, NL, SE)

1. Un composé de formule

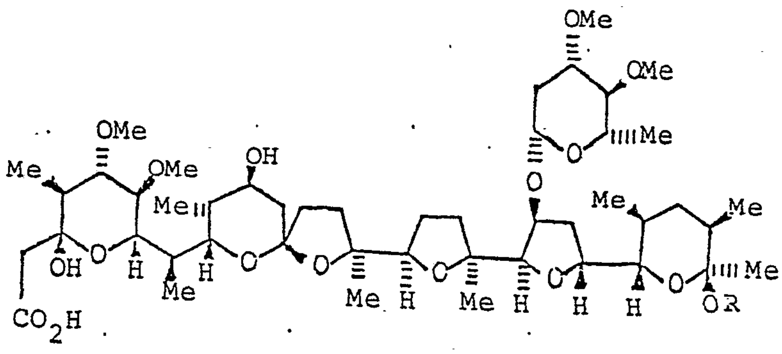

dans laquelle R représente un groupe alkyle en C 2-C 7 ou hydroxyalkyle en C 2-C 7, et ses sels acceptables pour l'usage pharmaceutique.

2. Un composé selon la revendication 1, dans lequel R représente un groupe éthyle, n-propyle, isopropyle, n-butyle, n-pentyle, n-hexyle, 1,2-dihydroxyéthyle ou 1,4-dihydroxybutyle et ses sels acceptables pour l'usage pharmaceutique.

3. Un composé selon la revendication 1, dans lequel R représente un groupe 1,3- ou 1,2-dihydroxypropyle, (S) ou (R)-3-hydroxybutyle ou 5-hydroxypentyle, et ses sels acceptables pour l'usage pharmaceutique.

4. L'éther éthylique de l'antibiotique X-14868A et ses sels acceptables pour l'usage pharmaceutique.

5. L'éther d'éthylène-glycol de l'antibiotique X-14868A et ses sels acceptables pour l'usage pharmaceutique.

6. L'éther n-hexylique de l'antibiotique X-14868A et ses sels acceptables pour l'usage pharmaceutique.

7. L'éther de 1,4-butylène-glycol de l'antibiotique X-14868A et ses sels acceptables pour l'usage pharmaceutique.

8. L'éther de 1,3-propane-diol de l'antibiotique X-14868A et ses sels acceptables pour l'usage pharmaceutique.

9. L'éther (S)-3-hydroxybutylique de l'antibiotique X-14868A et ses sels acceptables pour l'usage pharmaceutique.

10. L'éther (R)-3-hydroxybutylique de l'antibiotique X-14868A et ses sels acceptables pour l'usage pharmaceutique.

11. L'éther de 1,2-propane-diol de l'antibiotique X-14868A et ses sels acceptables pour l'usage pharmaceutique.

12. Un composé de formule

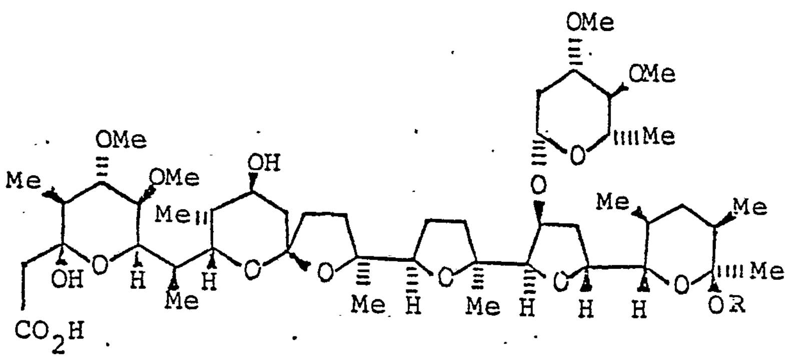

dans laquelle R représente un groupe alkyle en C 2-C 7 ou hydroxyalkyle en C 2-C 7 et ses sels acceptables pour l'usage pharmaceutique en tant qu'antibiotique.

13. Un composé selon la revendication 12 ou un sel acceptable pour l'usage pharmaceutique en tant qu'agent anticoccidiostatique.

14. Un composé selon la revendication 12 ou un sel acceptable pour l'usage pharmaceutique d'un tel composé en tant qu'agent antipaludéen.

15. Un composé selon la revendication 12 ou un sel acceptable pour l'usage pharmaceutique d'un tel composé en tant qu'agent améliorant la valorisation des aliments chez les ruminants et les porcs.

16. Procédé de préparation d'un composé de formule

(Siehe Schema Seite 17 f.)

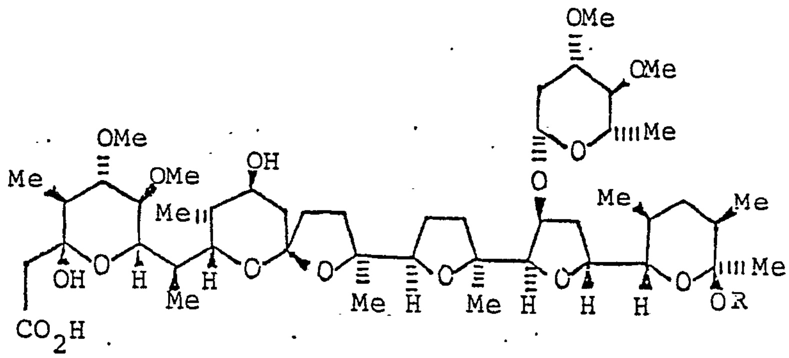

dans laquelle R représente un groupe alkyle en C 1-C 7 ou hydroxyalkyle en C 2-C 7, caractérisé en ce que l'on fait réagir un composé de formule

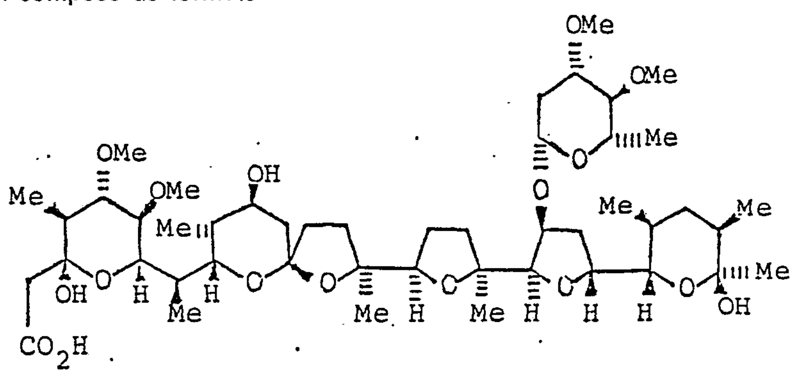

avec un alcool primaire ou secondaire en C 1-C 7 ou un alcool difonctionnel en C 2-C 7 en présence d'une résine échangeuse d'ions acides sous la forme $H^+$.

17. Antibiotique, caractérisé en ce qu'il contient un composé de formule

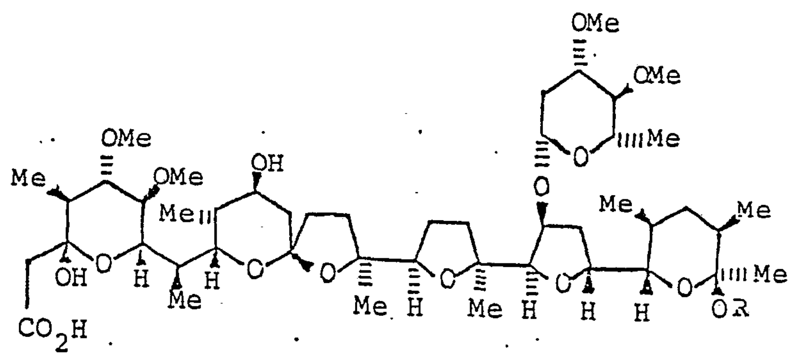

dans laquelle R représente un groupe alkyle en C 2-C 7 ou hydroxyalkyle en C 2-C 7, ou un sel acceptable pour l'usage pharmaceutique d'un tel composé, avec un véhicule.

18. Un agent anticoccidiostatique caractérisé en ce qu'il contient un composé répondant à la formule donnée dans la revendication 17 ou un sel acceptable pour l'usage pharmaceutique d'un tel composé, avec un véhicule.

19. Un agent antipaludéen caractérisé en ce qu'il contient un composé répondant à la formule donnée dans la revendication 17 ou un sel acceptable pour l'usage pharmaceutique d'un tel composé, avec un véhicule.

20. Un agent servant à améliorer la valorisation des aliments chez les ruminants ou les porcs, caractérisé en ce qu'il contient un composé répondant à la formule donnée dans la revendication 17 ou un sel acceptable pour l'usage pharmaceutique d'un tel composé, avec un véhicule.

21. L'utilisation d'un composé selon l'une des revendications 1 à 11, pour la préparation d'un antibiotique.

22. L'utilisation d'un composé selon l'une des revendications 1 à 11, pour la préparation d'un agent anticoccidiostatique.

23. L'utilisation d'un composé selon l'une des revendications 1 à 11, pour la préparation d'un agent antipaludéen.

24. L'utilisation d'un composé selon l'une des revendications 1 à 11, en tant qu'agent améliorant la valorisation des aliments chez les ruminants et les porcs.

**Revendications** (pour l'état contractant AT)

1. Procédé de préparation d'un composé de formule

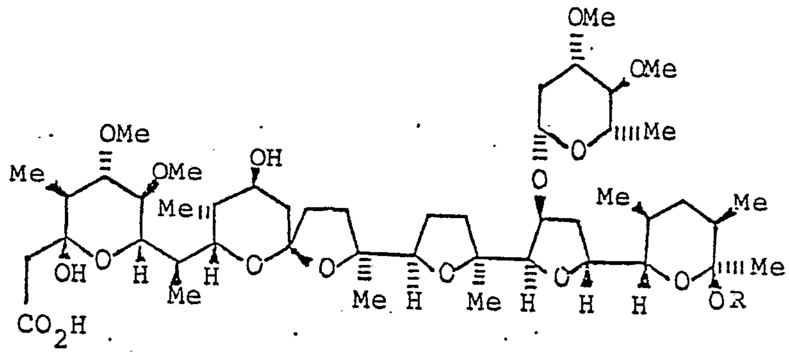

dans laquelle R représente un groupe alkyle en C 1-C 7 ou hydroxyalkyle en C 2-C 7, caractérisé en ce que l'on fait réagir un composé de formule

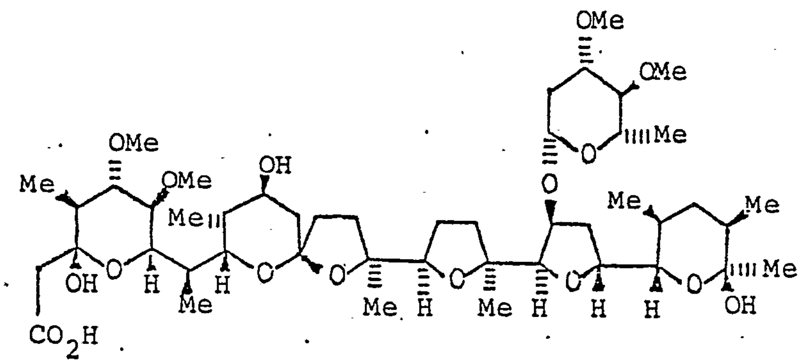

avec un alcool primaire ou secondaire en C 1-C 7 ou un alcool difonctionnel en C 2-C 7 en présence d'une résine échangeuse d'ions acides sous la forme $H^+$.

2. Procédé selon la revendication 1, caractérisé en ce que l'on prépare un composé de formule I ou un sel acceptable pour l'usage pharmaceutique d'un tel composé, dans lequel R représente un groupe éthyle, n-propyle, isopropyle, n-butyle, n-pentyle, n-hexyle, 1,2-dihydroxyéthyle ou 1,4-dihydroxybutyle.

3. Procédé selon la revendication 1, caractérisé en ce que l'on prépare un composé de formule I ou un sel acceptable pour l'usage pharmaceutique d'un tel composé, dans lequel R représente un groupe 1,3- ou 1,2-dihydroxypropyle, (S)- ou (R)-3-hydroxybutyle ou 5-hydroxypentyle.

4. Procédé selon la revendication 1, caractérisé en ce que l'on prépare l'éther éthylique de l'antibiotique X-14868A ou un sel acceptable pour l'usage pharmaceutique de cet éther éthylique.

5. Procédé selon la revendication 1, caractérisé en ce que l'on prépare l'éther d'éthylène-glycol de l'antibiotique X-14868A ou un sel acceptable pour l'usage pharmaceutique de cet éther.

6. Procédé selon la revendication 1, caractérisé en ce que l'on prépare l'éther n-hexylique de l'antibiotique X-14868A ou un sel acceptable pour l'usage pharmaceutique de cet éther.

7. Procédé selon la revendication 1, caractérisé en ce que l'on prépare l'éther de 1,4-butylène-glycol de l'antibiotique X-14868A ou un sel acceptable pour l'usage pharmaceutique de cet éther.

8. Procédé selon la revendication 1, caractérisé en ce que l'on prépare l'éther de 1,3-propanediol de l'antibiotique X-14868A ou un sel acceptable pour l'usage pharmaceutique de cet éther.

9. Procédé selon la revendication 1, caractérisé en ce que l'on prépare l'éther (S)-3-hydroxybutylique de l'antibiotique X-14868A ou un sel acceptable pour l'usage pharmaceutique de cet éther.

10. Procédé selon la revendication 1, caractérisé en ce que l'on prépare l'éther (R)-3-hydroxybutylique de l'antibiotique X-14868A ou un sel acceptable pour l'usage pharmaceutique de cet éther.

11. Procédé selon la revendication 1, caractérisé en ce que l'on prépare l'éther de 1,2-propanediol de l'antibiotique X-14868A ou un sel acceptable pour l'usage pharmaceutique de cet éther.